# EUROPEAN PATENT SPECIFICATION

(11) **EP 1 409 083 B1**
(45) Date of publication and mention of the grant of the patent: **05.12.2007**
(21) Application number: 00939235.8
(22) Date of filing: 31.05.2000
(51) Int. Cl.: A62D 3/00, A61L 2/06

(54) **DECONTAMINATION CONTAINER USEFUL, AMONG OTHER THINGS, FOR HIGHLY HAZARDOUS MATERIAL**
DEKONTAMINIERUNGSBEHÄLTER, U. A. GEEIGNET FÜR HOCHGEFÄHRLICHES MATERIAL
CONTENANT DE DECONTAMINATION UTILE NOTAMMENT POUR DES MATIERES TRES DANGEREUSES

(43) Date of publication of application: 21.04.2004
(73) Proprietor: JÄRVEN PLAST & SMIDE AKTIEBOLAG, S-891 60 Örnsköldsvik (SE)
(72) Inventor: NORDIN, Inga, 891 60 Örnsköldsvik (SE)
(74) Representative: Avellan-Hultman, Olle
(86) International application number: PCT/SE2000/001140
(87) International publication number: WO 2001/091858

(56) References cited:
- EP-A2- 0 262 451
- DE-A1- 3 421 719
- SE-C2- 510 465

## Description

The present invention relates to a decontamination container of the general type which is shown and described in the patent SE 510.564 and which is useful for decontamination of cloths and other objects which have been contaminated by chemical substances in the form of gases, liquids or particles, in particular bacteria, spores, virus and substances like highly hazardous waste for which there is a need for a very high temperature in order to kill the bacteria, the spores or viruses, or for decontamination of cloths and other objects.

The container is primarily intended for decontamination of cloths and objects which may need decontamination temperatures of between +78°C and +100°C, but it is also intended to be used for all kinds of other purposes like for warming up chilled persons and other objects, or for decontamination of living persons which have been exposed to hazardous substances or discharges, for decontamination of complete vehicles; as tempered field hospitals; as field saunas; as warmed staff service places, etc.

In simple cases decontamination of cloths and other objects can be made by flushing the objects with water or decontamination liquids, but in other cases the decontamination must be made using a hot environment, preferably hot air, sometimes hot air having an increased moisture percentage, or by a combination of liquid flushing and hot air treatment. For making it possible to execute a complete decontamination it is necessary that the cloths and other objects to be decontaminated are exposed to heat, preferably hot air of a very high temperature, in certain cases of up to +170°C. This is especially important in case of decontamination of cloths and other objects which have been exposed, for instance, to war substances like nerve gases, cholera bacteria, campylo bacterium, rabbit fever bacterium and other substances which are difficult to contaminate, and/or bacteria and virus etc. which are difficult to kill.

The decontamination apparatus which is described in the above mentioned patent SE 510.465 is useful for decontamination of cloths and other objects by means of air which when entering the apparatus may have a temperature of between +100°C and +130°C. Said temperature, however, is not sufficiently high for decontamination of certain chemical substances, war substances, certain types of bacteria and virus, hazardous waste from hospitals etc., what may need decontamination temperatures of as far as up to +170°C for neutralizing poisons and for killing bacteria and virus.

It has also shown that the temperature at the upper part of said priorly known decontamination apparatus, in which hot air enters at the lower part of the apparatus, is too low to provide the intended contamination since the temperature decreases while the air moves upwards thereby coming into contact with the relatively cooler cloths and products which have been hanged in the apparatus.

The patent publication DE 34 21 719 discloses an apparatus for decontamination of cloths, in which hot air is let into the apparatus from above into a container in which cloths are hanged. Said hot air entering from above leads to the disadvantage that the hot air is not distributed effectively to the lower areas of the apparatus, in particular since the hot air strives to rise in the container. For providing a distribution of the hot air as far as possible said apparatus comprises a suction pipe system at the lower part of the apparatus, and for making it possible to decontaminate cloths etc. the apparatus is equipped with a conveyor on which cloths are hanged, so that said cloths are moved along in the decontamination room. In said apparatus it is not possible to reach such high decontamination temperatures as up to +170°C as intended according to the present invention.

EP-A-0 262 451 discloses an apparatus in which the hot air is introduced through a wall at one side of a container and is sucked out through a wall at the opposite side of the apparatus. Also in this case the air will be cooled to a not acceptable low level during the passage horizontally through the decontamination container. Also not in said apparatus it is possible to reach the high decontamination temperatures of as far as up to +170°C which are needed for decontamination of certain types of bacteria, virus and chemical substances etc.

Most bacteria become killed at a temperature of +70-80°C, more specifically at about +78°C, certain other bacteria like rabbit fever bacterium, however, may need a killing temperature of at least +100-110°C, and this means that no part of the container should be allowed to keep a temperature which is less than said +100-110°C during the process of decontamination of bacteria. Temperatures of +30-42°C are extremely unsuitable, since bacteria grow extra strongly at said temperatures. Bacteria starts becoming killed at temperatures which are at least higher than about +60ºC. Therefore it is extremely important that the temperature at any point of the decontamination container can be raised to at least +110°C, or preferably as far as up to +170°C.

For providing an optimum distribution of warm air or hot air in the decontamination container the present invention suggests that hot air is introduced into the container through bores in an intermediate bottom at the floor level of the container and also through bores which are distributed over substantially the entire height of intermediate walls of the container, so that the hot air, at any place of the container, reaches the cloths or objects to be decontaminated, even at the uppermost parts, with substantially the same high temperature at all parts of the container.

A problem in the suggested apparatus for hot air decontamination is to reach such high temperature that is needed, especially temperatures of substantially more than +120°C, and as far as up to +170°C. This is a special problem at winter, when the temperature of the air entering the heater for providing the decontamination air can be very low, often as low as -20°C or still lower. In such case the heater is not capable or raising the temperature leaving the heater to the desired high temperature level. Attempts have been made to improve the flow of hot air using fans at the output end of the heater, but it has shown that such fans, or parts thereof, become damaged by the high temperatures from the air heater unit.

Therefore the present invention suggests that the apparatus be equipped with a preheater unit, which is preferably mounted at the exterior side of the decontamination container, and which raises the temperature of the air entering the main heater to +35-40ºC, and this makes it possible to reach the desired very high temperatures of the air which is circulated in the decontamination container.

It also has shown that an addition of water steam or superheated steam to the hot air entering the decontamination container can improve the transfer of heat to the goods to be decontaminated, and concurrently therewith the necessary time for performing the decontamination is substantially reduced.

For performing a fully acceptable hot air decontamination several different parameters have to be fulfilled, for instance:
- that the entire decontamination apparatus is formed as an integral unit containing all functional parts which are necessary for the decontamination,
- that the decontamination apparatus can be moved to any place where said apparatus is needed at the moment,
- that the air in the decontamination container can be brought to a temperature of at least +150°C, and for special purposes as far as up to +170°C,
- that said temperature can be maintained, upon need, for a long time, for instance for 5 hours or more,
- that the objects to be decontaminated are heated all throughout, what presupposes that the hot air is distributed both from underneath and from the sides and from above,
- that the decontamination can be executed by a combination of liquid decontamination, drying and hot air decontamination,
- that the decontamination is performed in closeable spaces or chambers, into which hot air (or another hot gas) can be introduced from underneath, from the sides and also from above and can be circulated in the chamber and can be drained at the top of the container,
- that the hot air is introduced in the chamber evenly distributed through several bores both in the bottom and in the sides, and eventually also in the ceiling,
- that the air entering the hot air producing unit, upon need can be preheated to at least +35-40°C and that the temperature of said air can be controlled and mixed in the best possible way.

When starting the decontamination of contaminated cloths or objects the temperature in the decontamination container successively increases from room temperature up to decontamination temperature, and it thereby happens that bacterium, virus and other things are let out through the air outlets of the decontamination container.

For preventing such outlet of contaminants in the ambient air the decontamination container therefore also ought to be formed with an effective filter at each outlet place for the decontamination air leaving the container, especially so called NBC (Nuclear-Biological-Chemical) filters.

When using the container for decontamination of living persons it is preferred that the container is formed with one or more shower units both acting from the ceiling and also from the side walls, so that a clean flushing can be made. When using the container as a war hospital, as a camp for the staff etc. it is important that the temperature and the moisture of the air can be kept on a suitable level, irrespective that the temperature and the moisture, for other purposes, can be varied within wide limits.

Thus, the invention relates to an apparatus for hot air decontamination of cloths, fabrics and all kinds of solid objects, and for many other purposes, at temperatures which can be varied within very wide limits, and which in special cases can be so high temperatures that are needed for decontamination and for neutralizing all kinds of chemical substances, for killing bacteria and virus and for many other types of decontamination, and which apparatus is formed as a closeable chamber, which at least at one place is formed with doors that can be opened and closed and which apparatus is heat insulated, and which includes one or more hot air units which are formed and arranged so that the hot air from this or these hot air units is lead down into an air passageway or a distribution chamber under, or behind, respectively, perforated plates at the bottom, at the sides and eventually also at the ceiling of the hot air chamber, through which perforated bores hot air flows out evenly distributed and leaves through outlet valves having effective filters at or adjacent the ceiling of the hot air chamber.

The hot air units can be of such known type which are commonly used as heaters in cars, buses or boats. For controlling the temperature of the hot air the air intakes to the heaters can be connected both the exterior air and also to the interior of the decontamination chamber, and the apparatus can contain one or more by thermostatically controlled shunt valves which foresee that the average temperature and the flow of hot air in the decontamination chamber is kept at a desired level. The distribution chamber for the hot air can be formed in that the bottom plate or panel and the side panels of the decontamination chamber are supported by spacer elements, and in that the hot air from the hot air unit or units is blown directly into the chamber through bores in the distribution chambers which are formed by the bottom plate and the side panels, respectively; the distribution bores may be of different size, and the total area of the distribution bores preferably can be made equal to the outlet area of the hot air unit or units.

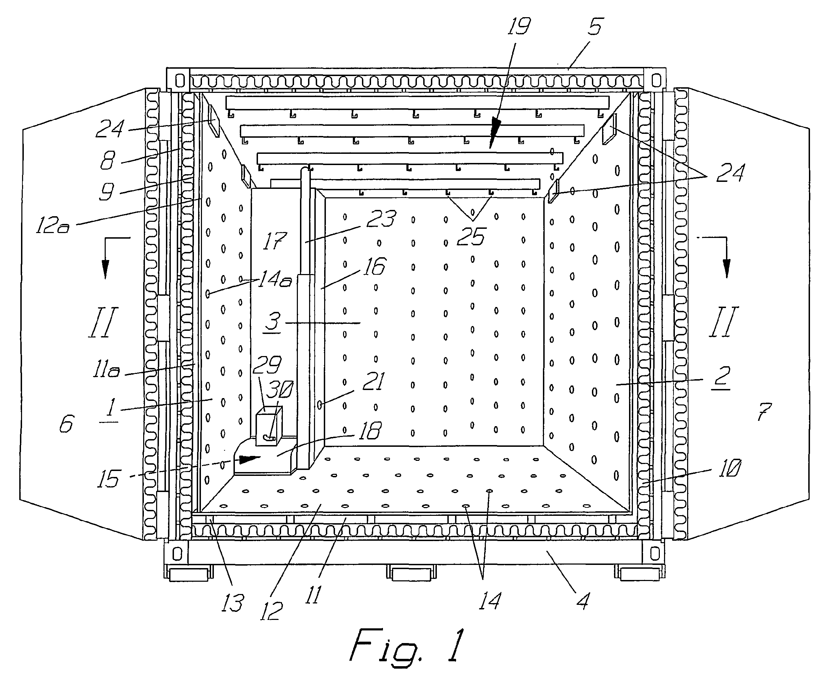

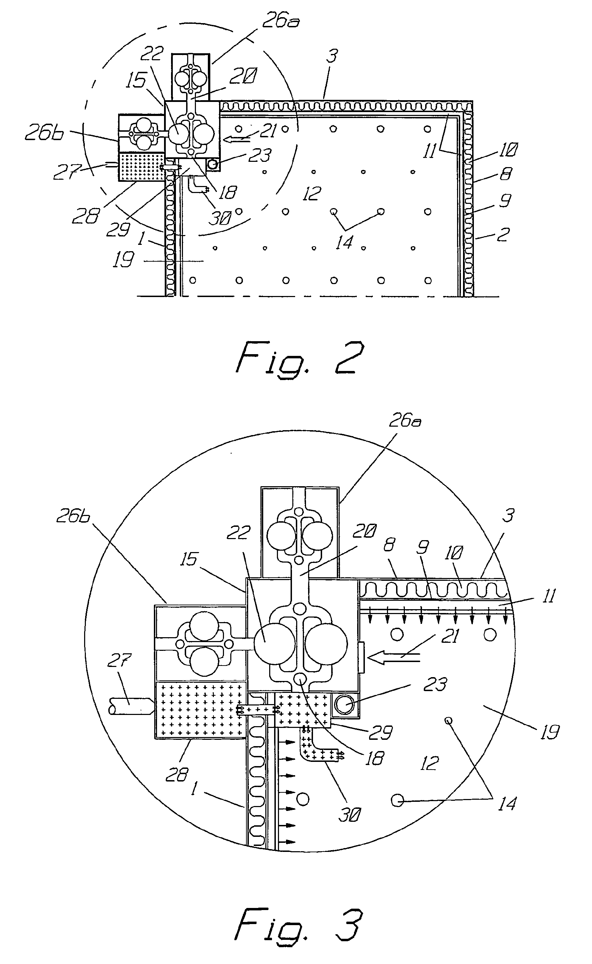

Further characteristics of the invention and advantages thereof will be evident from the following detailed specification in which reference is made to the accompanying drawings. In the drawings figure 1 is a central perspective view from outside and into a decontamination container according to the invention, as seen through open doors of the container. Figure 2 is a partial cross section along line II-II of figure 1, and figure 3 shows the encircled part of figure 3 in a larger scale.

The decontamination container shown in the drawings can have the same outer shape as an ordinary ship container. As known the container is parallelepipedic having longitudinal walls 1 and 2, a front end wall 3, floor 4, ceiling 5 and two doors 6, 7 which can be opened and mounted at the end of the container which is opposed to the end wall 3. The container is insulated at all sides and comprises an outer wall panel 8, an inner wall panel 9, which over small and thin spacer elements (not visible in the drawings) is kept at a predetermined distance from the outer wall panel 8. The intermediate space between the outer and inner wall panels 8, 9 is filled with an insulating material 10, for instance mineral wool.

Above the floor 4 there is an air distribution chamber 11, which is formed by an intermediate bottom plate or panel 12 which is supported at a certain distance above the inner panel 9 by several spacer blocks 13. The bottom plate 12 is formed with several through bores 14 which are distributed over the entire plate 12. The bores can be of the same or of different size and they also can be differently spaced so as to foresee that the hot air which is issued from the air distribution chamber 11 is distributed substantially equally and unitarily in the entire decontamination chamber.

In the same way as the bottom plate 12 the side walls 1, 2, 3 of the container, and preferably also the ceiling 5, are formed with an intermediate plate or panel 12a which delimits an air distribution chamber 11 a and which is formed with perforation bores 14a for blowing hot air into the contamination container over substantially the entire height thereof, and also from above into said decontamination container.

Any suitable hot air unit can be used for creating the hot air for the air distribution chamber. In the case illustrated in the drawings there is used one or two hot air units 15 of the type which is commonly used for instance i boats, trucks and buses etc., for instance the type of hot air unit named Eberspächer®, which have been connected parallelly to each other and have been mounted in a maneuvre box 16 at one corner of the decontamination chamber. Above the heaters there is a space 17 which is available from outside and which contains a self supply system comprising fuel tank for the heaters, batteries, connections for mains current, a motorized electric power station, means for controlling the temperatures and the flow of hot air to the air distribution chambers 11 and 11 a etc. In the illustrated case the hot air units 15 are mounted lying in the space 17 and so that the hot air, over a hot air passageway 18, is lead down through the bottom plate 12 and the wall plates 12a into the air distribution chambers 11 and 11 a and from there through the bores 14 of the bottom plate 12 and the bores 14a of the side plates 12a, and in the actual case in the ceiling plate and is distributed in the entire decontamination space 19.

For creating the best possible distribution of hot air for certain purposes it can be suitable to mix cold air from a cold air inlet 20 at the exterior side of the decontamination container with hot air from a hot air inlet 21 in the decontamination space 19. By a suitable mixing of air 20 entering the hot air units 15 with hot return air 21 the incoming mixed air will have a temperature of for instance +50°C and the outlet hot air will have such temperature that the average temperature in the decontamination space is between +130°C and +170°C.

The mixing of cold air from the cold air inlet 20 and hot air from the hot air inlet 21 is preferably controlled by a shunt system 22 which is connected to the thermostatical control means for the apparatus. The exhaust gases from the hot air units 15 leave through a chimney 23 opening at the roof of the decontamination container.

The hot air leaving the air distribution chambers 11 and 11 a through the distribution bores 14 of the intermediate bottom 12 and the wall panels 12a moves upwards and sideways through the decontamination space 19 and leave through several outlet valves 24 provided at or adjacent the ceiling of the container, preferably provided adjacent the side walls 1 and 2. The valves preferably are formed with choke flies, so that the pressure of the hot air in the decontamination space 19 can be varied as desired, and so that cold air does not enter the decontamination space through the valves. The valves 24 drain both circulated air and moisture from the decontamination space. For preventing outlet of bacteria, virus and contaminants a filter is mounted in connection to the air outlet valve 24, for instance a NBC (Nuclear-Biological-Chemical) filter, which effectively filters off bacteria and contaminants. This is especially important before the temperature of the container has reached such temperature that bacteria and virus become killed.

For hanging of contaminated cloths or other objects there are several hooks 25 provided in the ceiling of the decontamination space, in which hooks a large amount of cloths and other objects can be hanged. It is also possible to place objects on the intermediate bottom floor plate 12.

For providing the very high air temperature which in necessary for neutralizing poisons, war substances, war gases, different types of bacteria and virus etc., and which temperature in certain cases must be as high as + 170°C it may be necessary to use a preheater for the air entering the hot air units 15. This is especially actual at winters or otherwise in a cold climate. To this end the apparatus can be formed with one or more preheater units 26a, 26b which are mounted at the exterior side of the decontamination container, and which upon need can be connected directly to the main heaters 15 and which foresees or foresee that the temperature of the air entering the main heaters 15 is at least between +35°C and +40°C.

For further improving the heat distribution, and for reducing the decontamination time, there is a system for supply of overheated steam to the decontamination air in the decontamination space 19. Said system can be formed with one or more nozzles 27 creating finely distributed drops of water which are preheated in a steam treatment container 28 provided adjacent any of the heater units 15, 26 and from which steam is blown out by means of a fan (not shown) through a distribution chamber 29 and into the decontamination space 19 through one or more steam pipes 30.

The container is formed so that the entire vehicle can be driven into same for being decontaminated.

For decontamination of living persons and animals it is important that the temperature and the moisture is kept on a suitable level, and that the container inside same is formed with one or more shower units mounted both in the ceiling and along the walls of the container so that shower decontamination can be made without the assistance of qualified personnel.

The described apparatus allows a decontamination of all kinds of different objects made of any type of material, and also drying of objects which have been contaminated by liquids or which have been washed in water or have been clean flushed by means of water or another decontamination liquid. Since the decontamination container is self supporting as concerns fuel, electricity and other necessities it can be transported by truck, by railway, by boat or by airplane or helicopter to any place where decontamination ought to be made. The decontamination container also can be used for transportation of goods, and to this end it may be formed with suitable clamp eyelets (not shown) at the bottom plate, at the walls or in the ceiling.

After hanging of the goods to be decontaminated in the hooks 25 in the ceiling of the decontamination space 19, or after the goods to be decontaminated have been placed on the intermediate bottom plate 12 the doors 6 and 7 are closed, and the hot air units 15 are started from outside the maneuvre box 16. After full heat has been reached of the hot air in the container space 19, what can take another 5-10 minutes, the hot air decontamination process is started, and said hot air treatment may take between one and five hours depending on what objects are being decontaminated. The decontamination also can take place during transportation of the decontamination apparatus between different places.

As known all conduits, existing light cables etc. are shock and shaking proofly mounted and are mounted under water proof conditions, for instance enclosed under the inner wall panels 9, so that the decontamination space 19 can be flushed upon need. The decontamination container, likewise as known, can be formed with an access ramp on which trucks can drive up and deliver goods in the decontamination space, or on which vehicles to be decontaminated can drive into the space.

Upon decontamination of certain types of substances, in particular gases, war substances, hazardous products from hospitals etc. it is preferable that filters (not shown) are arranged in connection to the outlet valves 24 of the decontamination container.

### REFERENCE NUMERALS

- 1: side wall
- 2: side wall
- 3: end wall
- 4: floor
- 5: ceiling
- 6: door
- 7: door
- 8: outer wall panel
- 9: inner wall panel
- 10: insulation material
- 11: air distribution chamber (11a)
- 12: intermediate bottom plate (12a)
- 13: spacer block
- 14: perforation bore (14a)
- 15: hot air unit (main heater)
- 16: maneuvre box
- 17: space
- 18: hot air passageway
- 19: decontamination space
- 20: cold air inlet
- 21: hot air inlet
- 22: shunt system
- 23: chimney
- 24: outlet valve
- 25: hook
- 26: preheater unit (26a, 26b)
- 27: nozzle
- 28: steam treatment container
- 29: distribution chamber
- 30: steam pipe

## Claims

1. Container for hot air decontamination of human beings, animals, cloths and other objects which have been contaminated by chemical substances in the form of gases, liquids, radioactive fallout or particles, and also for decontamination of highly hazardous material and cloths and objects which have been contaminated by bacteria, virus, nerve gas, cholera bacterium, rabbit plague and other substances, like bacteria and virus etc. which are difficult to kill, and comprising a decontamination container (1-7) which can be opened and closed by doors (6, 7) and which is formed with a decontamination space (19) adapted to be supplied with decontamination air of high temperature, and in which the material to be decontaminated is adapted to be hanged or placed, **characterized in**
**that** the apparatus is formed with an intermediate panel (12, 12a) both at the bottom (4) and along its walls (8, 9) and eventually also over the ceiling (5), which panel (12, 12a) is mounted spaced by spacer elements (13) from the floor (4) and the walls (8, 9) and the ceiling (5), respectively, of the container,
**that** the space between the intermediate panel (12, 12a) and the bottom (4) and the walls (1, 2, 3), respectively, of the container provides an air distribution chamber (11, 11a) into which hot air is entered,
and **that** the intermediate panels (12, 12a) are formed with several through bores (14, 14a) through which hot air is given off from the air distribution chambers (11, 11a) to the decontamination space (19),
which bores (14) are arranged so that hot air which is given off from the air distribution chambers (11) is distributed substantially equally and unitarily in the entire decontamination space (19).

2. Decontamination container according to claim 1, **characterized in that** it is formed with one or more main hot air units (15) which provide hot air to all air distribution chambers (11, 11 a), and one or more supplementary heater units (26) for preheating of the air entering said one or more main hot air apparatus (15).

3. Decontamination container according to claim 2, **characterized in that** the supply air for said one or more main hot air units (15) is received both from the ambient outer air (20) or the supplemental heater unit (26) and from the inner of the decontamination space (19), and **in that** the apparatus is formed with a thermostatically controlled shunt system (22) for mixing of hot and cold air for giving the supply air to said one or more main hot air units (15) a specifically desired temperature.

4. Decontamination container according to claim 1, 2 or 3, **characterized in that** total area of the bores (14, 14a) of the intermediate panels (12, 12a) substantially corresponds to the outlet area of the hot air passageway (18) from said one or more main hot air units (15).

5. Decontamination container according to any of the preceding claims, **characterized in that** the container is formed with outlet valves (24) for draining of consumed hot air and provided at or adjacent the ceiling (5) of the decontamination container and close the side walls (1, 2, 3), which outlet valves (24) include filters for separating off contaminants and waste products from the outlet air.

6. Decontamination container according to any of the preceding claims, **characterized in that** it comprises two parallelly mounted main hot air units (15) which give off their hot air directly into the air distribution chambers (11, 11a) behind the intermediate panels (12, 12a), and **in that** supplemental air connections to the main hot air unit (15) or units are connected to one or more supplemental heater units (26a, 26b), and **in that** the container comprises a thermostatically controlled shunt system (22) providing a mixing of cold ambient air and hot air from the inner of the decontamination space (19), which mixed air entering the main hot air units (15) has a temperature of for instance +50°C.

7. Decontamination container according to any of the preceding claims, **characterized in that** said one or more main hot air units (15), together with one or more supplementary heater units (26), is/are adapted to supply air to the decontamination space (19) which has a temperature of more then +130°C, preferably or up to +170°C.

8. Decontamination container according to any of the preceding claims, **characterized in that** the decontamination container is formed with means (27-30) for supplying water steam or superheated steam to the decontamination space (19) together with hot air.

9. Decontamination container according to any of the preceding claims, **characterized in that** it includes a self supply system (16, 17) comprising a fuel operated electric power station, a fuel tank for supplying fuel to the hot air units (15, 26), a coupling panel, a supervising panel for temperature and flow of air to and from the hot air units, a thermostat for the hot air entering the decontamination space, a system for shunting (22) of hot and cold air for the supply air to the hot air units.

## Patentansprüche

1. Behälter für die Heißluftdekontaminierung von Menschen, Tieren, Bekleidungsstücken und anderen Objekten, die kontaminiert wurden durch chemische Substanzen in Form von Gasen, Flüssigkeiten, radioaktivem Niederschlag oder Partikeln und auch für die Dekontaminierung von hochgefährlichem Material und Bekleidungsstücken und Objekten, die von Bakterien, Viren, Nervengas, Cholerabakterien, Kaninchenpest und anderen Stoffen wie Bakterien und Vieren etc. kontaminiert wurden, die schwer abzutöten sind, und umfassend einen Dekontaminierungsbehälter (1-7), der über Türen (6, 7) geöffnet und geschlossen werden kann und der mit einem Dekontaminierungsraum (19) ausgebildet ist, der ausgelegt ist, um mit Dekontaminierungsluft hoher Temperatur versorgt zu werden und in den das zu dekontaminierende Material aufgehängt oder plaziert werden kann, **dadurch gekennzeichnet,**
**daß** die Vorrichtung mit einem Zwischenpaneel (12, 12a) sowohl an dem Boden (4), wie auch entlang ihrer Wände (8, 9) und eventuell auch über die Decke (5) ausgebildet ist, wobei das Paneel (12, 12a) über Abstandshalterelemente (13) von dem Boden (4) bzw. den Wänden (8, 9) bzw. der Decke (5) des Behälters beabstandet montiert ist,
**daß** der Raum zwischen dem Zwischenpaneel (12, 12a) bzw. dem Boden (4) bzw. den Wänden (1, 2, 3) des Behälters eine Luftverteilungskammer (11, 11a) liefert, in die Heißluft eingeführt wird, und daß die Zwischenpaneele (12, 12a) mit mehreren Durchgangsbohrungen (14, 14a) ausgebildet sind, durch die Heißluft von den Luftverteilungskammern (11, 11a) zu dem Dekontaminierungsraum (19) abgebbar ist,
wobei die Bohrungen (14) derart angeordnet sind, daß von den Luftverteilungskammern (11) abgegebene Heißluft im wesentlichen gleichmäßig und einheitlich in dem gesamten Dekontaminierungsraum (19) verteilt wird.

2. Dekontaminierungsbehälter nach Anspruch 1, **dadurch gekennzeichnet, daß** er mit einer oder mehreren Hauptheißlufteinheiten (15) ausgebildet ist, die allen Luftverteilungskammern (11, 11a) Heißluft liefern, und einer oder mehreren Zusatzheizeinheiten (26) zum Vorheizen der Luft, die in eine oder mehrere Hauptheißluftvorrichtungen (15) eintritt.

3. Dekontaminierungsbehälter nach Anspruch 2, **dadurch gekennzeichnet, daß** die Zuführluft für die eine oder mehreren Hauptheißlufteinheiten (15) sowohl von der äußeren Umgebungsluft (20) oder der Zusatzheizeinheit (26), wie auch dem Inneren des Dekontaminierungsraums (19) empfangen wird, und daß die Vorrichtung mit einem thermostatisch gesteuerten Abzweigsystem (22) ausgebildet ist zum Mischen von heißer und kalter Luft, um der Zuführluft zu der einen oder den mehreren Hauptheißlufteinheiten (15) eine speziell gewünschte Temperatur zu verleihen.

4. Dekontaminierungsbehälter nach Anspruch 1, 2 oder 3, **dadurch gekennzeichnet, daß** die gesamte Fläche der Bohrungen (14, 14a) der Zwischenpaneele (12, 12a) im wesentlichen der Auslaßfläche des Heißluftdurchgangs (18) von der einen oder den mehreren Hauptheißlufteinheiten (15) entspricht.

5. Dekontaminierungsbehälter nach einem der vorstehenden Ansprüche, **dadurch gekennzeichnet, daß** der Behälter mit Auslaßventilen (24) ausgebildet ist zum Ablassen verbrauchter Heißluft, die bei oder angrenzend an der Decke (5) des Dekontaminierungsbehälters und in der Nähe der Seitenwände (1, 2, 3) vorgesehen sind, wobei die Auslaßventile (24) Filter aufweisen zum Abtrennen von Kontaminierungen und Abfallprodukten aus der Auslaßluft.

6. Dekontaminierungsbehälter nach einem der vorstehenden Ansprüche, **dadurch gekennzeichnet, daß** er zwei parallel montierte Hauptheißlufteinheiten (15) umfaßt, die ihre Heißluft direkt in die Luftverteilungskammern (11, 11a) hinter den Zwischenpaneelen (12, 12a) abgeben, und daß die zusätzlichen Luftverbindungen zu der Hauptheißlufteinheit (15) oder -einheiten mit einer oder mehreren zusätzlichen Heizeinheiten (26a, 26b) verbunden sind, und daß der Behälter ein thermostatisch gesteuertes Abzweigsystem (22) umfaßt, das ein Gemisch aus kalter Umgebungsluft und heißer Luft von dem Inneren des Dekontaminierungsraums (19) liefert, wobei das in die Hauptheißlufteinheiten (15) eintretende Luftgemisch eine Temperatur von beispielsweise +50°C hat.

7. Dekontaminierungsbehälter nach einem der vorstehenden Ansprüche, **dadurch gekennzeichnet, daß** die eine oder mehrere Hauptheißlufteinheiten (15), zusammen mit einer oder mehreren Zusatzheizeinheiten (26) ausgelegt ist/sind, um dem Dekontaminierungsraum (19) Luft zuzuführen, die eine Temperatur von mehr als +130°C, vorzugsweise von bis zu +170°C hat,

8. Dekontaminierungsbehälter nach einem der vorstehenden Ansprüche, **dadurch gekennzeichnet, daß** der Dekontaminierungsbehälter ausgebildet ist mit Mitteln (27-30) zur Zufuhr von Wasserdampf oder Heißdampf zu dem Dekontaminierungsraum (19) zusammen mit Heißluft.

9. Dekontaminierungsbehälter nach einem der vorstehenden Ansprüche, **dadurch gekennzeichnet, daß** er ein selbstversorgendes System (16, 17) aufweist, das ein Brennstoff-betriebenes Elektrizitätswerk, einen Brennstofftank zur Zufuhr von Brennstoff zu den Heißlufteinheiten (15, 26), ein Kopplungspaneel, ein Überwachungssteuerpult für Temperatur und Luftstrom zu und von den Heißlufteinheiten, einen Thermostaten für die in den Dekontaminierungsraum eintretende Heißluft, ein System zum Abzweigen (22) heißer und kalter Luft für die Zufuhrluft zu den Heißlufteinheiten, umfaßt.

## Revendications

1. Contenant de décontamination par de l'air chaud d'êtres humains, d'animaux, de vêtements et d'autres objets qui ont été contaminés par des substances chimiques sous la forme de gaz, liquides, particules ou déchets radioactifs, et également pour la décontamination de matériels hautement dangereux et des vêtements et objets qui ont été contaminés par des bactéries, des virus, du gaz neurotoxique, la bactérie du choléra, la peste du lapin et d'autres substances, telles que bactéries et virus etc., qui sont difficiles à éliminer, et comprenant un contenant de décontamination (1-7) qui peut être ouvert ou fermé par des portes (6, 7) et qui est formé d'un espace de décontamination (19) adapté pour être approvisionné en air de décontaminatison à haute température et dans lequel le matériel à décontaminer peut être suspendu ou placé, **caractérisé en ce que**
■ le dispositif est construit avec un panneau intermédiaire (12, 12a), à la fois sur le fond (4) et le long de ses parois (8, 9) et éventuellement aussi sur le dessus (5), lequel panneau (12, 12a) est monté, espacé par des éléments de séparation (13), du dessous (4) et des parois (8, 9) et du dessus (5), respectivement, du contenant,
■ l'espace entre le panneau intermédiaire (12, 12a) et le fond (4), et les parois (1, 2 3), respectivement, du contenant, forme une chambre de distribution d'air (11, 11a) dans laquelle on fait rentrer de l'air chaud, et **en ce que**
■ les panneaux intermédiaires (12, 12a) sont construits avec plusieurs perforations (14, 14a) par lesquelles de l'air chaud est dégagé à partir des chambres de distribution d'air (11, 11a) vers l'espace de décontamination (19),
lesquelles perforations (14) sont arrangées de manière à ce que l'air chaud qui est dégagé des chambres de distribution d'air (11), soit distribué sensiblement de manière égale et unitaire dans l'espace entier de décontamination (19).

2. Contenant de décontamination selon la revendication 1, **caractérisé en ce qu'**il est formé d'une ou de plusieurs unités principales d'air chaud (15) qui fournissent de l'air chaud à toutes les chambres de distribution d'air (11, 11a), et d'une ou plusieurs unités de chauffage supplémentaires (26), pour le préchauffage de l'air entrant dans ledit dispositif principal d'air chaud (15) ou dans plusieurs dispositifs (15).

3. Contenant de décontamination selon la revendication 2, **caractérisé en ce que** l'approvisionnement d'air de ladite une ou plusieurs unités principales d'air chaud (15) est reçu à la fois, à partir de l'air extérieur ambiant (20) ou de l'unité de chauffage supplémentaire (26), et à partir de l'intérieur de l'espace de décontamination (19), et **en ce que** l'appareillage est constitué d'un système de shunt (22), contrôlé thermostatiquement, pour mélanger l'air chaud et l'air froid afin d'approvisionner en air ladite une ou à plusieurs unités principales d'air chaud (15) à la température spécifiquement désirée.

4. Contenant de décontamination selon la revendication 1, 2 ou 3, **caractérisé en ce que** la surface totale des perforations (14, 14a) des panneaux intermédiaires (12, 12a) correspond sensiblement à l'aire de sortie de l'évacuation d'air chaud (18) dudit ou desdites plusieurs unités principales d'air chaud (15).

5. Contenant de décontamination selon l'une quelconque des revendications précédente, **caractérisé en ce que** le contenant est construit avec des valves de sortie (24) pour drainer l'air chaud consommé et disposées sur le dessus (5) du contenant de décontamination ou de manière adjacente adjacentes à et à proximité des parois latérales (1,2,3), lesquelles valves de sortie (24) comportent des filtres pour séparer les contaminants et les produits de déchet de l'air de sortie.

6. Contenant de décontamination selon l'une quelconque des revendications précédentes, **caractérisé en ce qu'**il comprend deux unités principales d'air chaud (15) montées parallèlement, qui délivrent leur air chaud directement dans les chambres de distribution d'air (11, 11a), derrière les panneaux intermédiaires (12, 12a), et **en ce que** les connections d'air supplémentaires à l'unité principale d'air chaud (15) ou aux unités, sont connectées à une ou plusieurs unités de chauffage supplémentaires (26, 26a), et **en ce que** le contenant comprend un système de shunt (22) contrôlé thermostatiquement délivrant un mélange d'air ambiant froid et d'air chaud venant de l'intérieur de l'espace de décontamination (19), lequel air mélangé entrant dans les unités principales d'air chaud (15), a une température de par exemple + 50°C.

7. Contenant de décontamination selon l'une quelconque des revendications précédentes, **caractérisé en ce que** ladite ou lesdites plusieurs unités principales d'air chaud (15), ensemble avec une ou plusieurs unités de chauffage supplémentaires (26) est/sont aptes à délivrer à l'espace de décontamination (19) de l'air qui a une température supérieure à 130°C, de préférence ou jusqu'à + 170°C.

8. Contenant de décontamination selon l'une quelconque des revendications précédentes, **caractérisé en ce que** le contenant de décontamination est construit avec des moyens (27-30) pour fournir de la vapeur d'eau ou de la vapeur d'eau surchauffée à l'espace de décontamination conjointement avec de l'air chaud, (19).

9. Contenant de décontamination selon l'une quelconque des revendications précédentes, **caractérisé en ce qu'**il comprend un système autonome d'alimentation (16, 17) comprenant un groupe électrogène fonctionnant au fuel, un réservoir de fuel pour alimenter en fuel les unités d'air chaud (15, 26), un panneau de couplage, un panneau de pilotage pour la température et le débit de l'air allant vers et provenant des unités d'air chaud, un thermostat pour l'air chaud entrant dans l'espace de décontamination, un système (22) pour dériver l'air chaud et froid de l'air d'alimentation vers les unités d'air chaud.
